# EUROPEAN PATENT APPLICATION

(11) **EP 1 749 828 A1**
(43) Date of publication of application: **07.02.2007**
(21) Application number: 05381040.4
(22) Date of filing: 04.08.2005
(51) Int. Cl.: C07D 403/10

(54) **Process for preparing an angiotensin II receptor antagonist**

(71) Applicant: FARMAPROJECTS S.L., 08302 L'Hospitalet del Llobregat (ES)
(72) Inventor: Bessa, Jordi, 08004 Barcelona (ES)
(74) Representative: Barlocci, Anna

(57) **Abstract**

Process for preparing irbesartan, protected forms for the preparation thereof, or a pharmaceutically acceptable salt thereof, that comprises the reaction between a biphenylamino derivative and an oxazolone derivative.

## Description

### FIELD OF THE INVENTION

The present invention relates to a process for preparing an angiotensin II receptor antagonist, in particular irbesartan, and protected forms for the preparation thereof.

### BACKGROUND ART

Irbesartan is an angiotensin II receptor antagonist of formula:

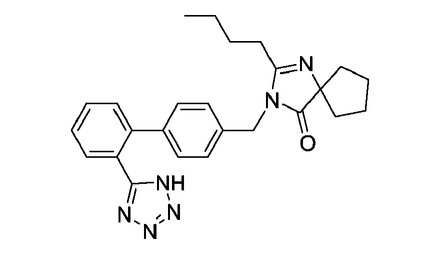

Angiotensin II is a peptide hormone that is a potent vasopressor. It is the biologically active product of the renin-angiotensin system. Renin acts on the angiotensinogen of the plasma to produce angiotensin I, which is converted to angiotensin II by the action of the angiotensin I converting enzyme.

Irbesartan inhibits the action of angiotensin II on its receptors and thus prevents the increase in blood pressure produced by the hormone-receptor interaction. It is therefore useful in the treatment of hypertension and heart failure.

Several synthetic routes have been described in the literature for the preparation of irbesartan. Most of the routes comprise the reaction between a bromomethylbiphenyl or an aminomethylbiphenyl compound with 2-butyl-1,3-diazaspiro[4,4]non-1-en-4-one (e.g. EP454511, WO2005051943, WO9906398, WO2004007482). The last step of the processes described in said first three patent applications corresponds to the formation of the tetrazole ring from a cyano group employing an azide derivative.

There have also been described processes for preparing irbesartan in which the last step corresponds to the formation of the biphenyl moiety. Thus, WO2004065383A2 describes a process for preparing irbesartan by a Suzuki coupling reaction comprising the reaction of a bromobenzyl spiro compound with a 2-(tetrazol-5-yl)phenylboronic acid derivative in the presence of a palladium catalyst and triphenyl phosphine in 1,2-dimethoxyethane (DME) and tetrahydrofuran.

WO2004072064A1 discloses different routes for the synthesis of irbesartan, in which the last step is the formation of the spiro cycle, followed by deprotection. The formation of the spirocycle is described:
- By reaction of 1-pentanamidocyclopentanecarboxamide with 5-(4'-bromomethylbiphenyl-2-yl)-trityl-1*H*-tetrazole,
- by reaction of an amino compound with an imidate intermediate, under inert atmosphere in dry toluene,
- by reaction of a valeramide derivative in the presence of oxalyl chloride and 2,6-lutidine with an amine under argon.

### SUMMARY OF THE INVENTION

The problem to be solved by the present invention is to provide an efficient alternative process for preparing irbesartan.

The solution is based on that the present inventors have identified a simplified process for preparing irbesartan. Said process comprises the reaction between an oxazolone and a primary amine, in particular, between the spiro compound 2-butyl-3-oxa-1-azaspiro[4.4]non-1-en-4-one and an aminomethylbiphenyl intermediate. Said spiro compound has only been found to be described in an article of 1966 (c.f. Winters, G. et al., Farmaco, Edizione Scientifica (1966), 21 (9), 624-30). Surprisingly its use for the manufacture of irbesartan, according to the process of the invention, has been found to be advantageous. See working examples 1-5 herein for a further description.

Accordingly, a first aspect of the invention relates to a process for preparing a compound of formula (I) or a pharmaceutically acceptable salt thereof 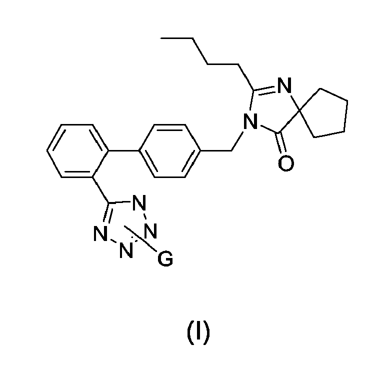 wherein:
G is H or a tetrazole protecting group,
   comprising the reaction between an intermediate of formula (II) or an acid addition salt thereof 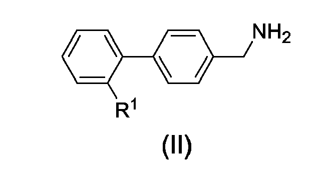
wherein:
R¹ is a tetrazolyl group or an intermediate or protected form that can be transformed to a tetrazolyl group
   and an intermediate of formula (III)

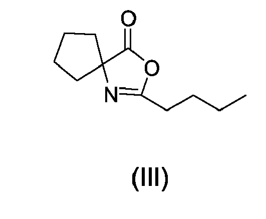

in an appropriate solvent system and thereafter as necessary transforming said intermediate or protected forms of R¹ to a tetrazolyl group and, if desired, converting said compound of formula (I) to a pharmaceutically acceptable salt thereof.

The process of the present invention presents several advantages that are important for the manufacture of irbesartan on an industrial scale. It is a simplified process that renders irbesartan in one step from intermediates easily to obtain from commercial products. The reaction is selective for the primary amine, and presents no interaction with the NH group of the tetrazole ring. Thus, advantageously, the reaction proceeds with high yields even if no protecting groups (e.g. the trityl group for protecting the tetrazole ring) are employed.

Advantageously, when the process is carried out without protecting groups, additional steps of protection and deprotection are avoided and also the need of inflating the mass, which has to be subsequently deflated. This atomic economy is an important advantage for a manufacturing method at industrial scale.

A further advantage of the process is that irbesartan may be obtained from commercial products without the need of handling explosive and highly toxic reagents, such as azide derivatives.

### Definitions

Acid addition salts of compounds of formula (II) or of formula (V) refer to amino salts formed with inorganic and organic acids such hydrochlorides, hydrobromides, sulphates, nitrates, phosphates, organic sulfonates, among others.

By an intermediate form that can be transformed to a tetrazolyl group it is understood a group such as a cyano group, that can be transformed to a tetrazolyl group by methods well known to the skilled in the art.

By a protected form that can be transformed to a tetrazolyl group it is understood in the present invention, a tetrazole ring protected with a tetrazole protecting group.

By a leaving group X, it is meant in the present invention, a good leaving group in the conditions of a Suzuki coupling, such as an atom of Cl, Br, I, or a trifluoromethanesulfonyloxy group

By a C₁-C₆ linear or branched alkyl in the present invention it is meant a linear or branched alkyl group which contains up to 6 carbon atoms. Thus it includes, for instance, methyl, ethyl, propyl, isopropyl, butyl, isobutyl, sec-butyl, *tert*-butyl, n-pentyl, 1,2-dimethyl propyl, 1,1-dimethyl propyl, 2,2-dimethyl propyl, 2-ethyl propyl, n-hexyl, 1,2-dimethyl butyl, 2,3-dimethyl butyl, 1,3-dimethylbutyl, 1-ethyl-2-methylpropyl, and 1-methyl-2-ethyl propyl groups.

By a (C₁-C₄)-alkoxy in the present invention it is meant a linear or branched alkoxy group which contains up to 4 carbon atoms. Thus it includes, for instance, methoxy, ethoxy, propoxy, isopropoxy, butoxy, isobutoxy, sec-butoxy and *tert*-butoxy groups.

### DETAILED DESCRIPTION OF PARTICULAR EMBODIMENTS

As described above, in the process according to the first aspect of the invention, intermediate of formula (III) may react with an intermediate of formula (II) or an acid addition salt thereof. Preferably, intermediate of formula (III) is reacted with intermediate of formula (II), or its hydrochloride.

The best conditions to carry out the process vary according to the parameters considered by the person skilled in the art, such as the solvents, temperature and similar. Such reaction conditions may be easily determined by the person skilled in the art by routine tests, and with the teaching of the examples included in this document.

The reaction may be carried out in different solvents systems. Preferably, the solvent system is an organic solvent or a mixture of organic solvents. The organic solvent may be selected from aliphatic or aromatic (C₆-C₈) hydrocarbons such as toluene, xylene; aliphatic ethers such as dimethoxyethane, diethoxymethane, diglyme, dioxane, and tetrahydrofuran, and aliphatic (C₁-C₅) alcohols such as ethyl, propyl, isopropyl, butyl, isobutyl, tert-butyl, pentyl, isopentyl and tert-pentyl alcohol; ketones such as acetone, methylethylketone, or a polar aprotic solvent. Preferably, the solvent system comprises a polar aprotic solvent, since, especially when R¹ is H, the reaction proceeds faster when it is carried out in presence of a polar aprotic solvent. Polar aprotic solvents that may be suitable for the reaction include: N-dialkylated amides such as *N,N*-dimethylformamide (DMF), 1-methylpyrrolidone (NMP), *N,N*-dimethylacetamide (DMA), dioxane and dimethyl sulfoxide (DMSO). In a preferred embodiment, the reaction is carried out in presence of DMF or NMP.

The reaction between the intermediate of formula (II) or an acid addition salt thereof and intermediate of formula (III) is preferably carried out in a neutral medium or in the presence of an acid catalyst. By neutral medium it is understood a medium without the presence of any acidic or basic agent. In a preferred embodiment, it is carried out in the presence of an acid catalyst, since, advantageously, it generally leads to higher yields. Suitable acid catalysts include: inorganic acids such as hydrochloric acid, hydrobromic acid, phosphoric acid, sulfuric acid, nitric acid and boric acid; organic sulfonic acids such as methanesulfonic acid, p-toluenesulfonic acid, camphorsulfonic acid, pyridinium p-toluenesulfonate, etc; and Lewis acids such as aluminum trichloride, boron trifluoride, zinc dichloride, tin tetrachloride, etc. In a more preferred embodiment, the acid catalyst is selected from the group consisting of methanosulfonic acid, *p*-toluensulfonic acid and hydrochloric acid.

In a preferred embodiment, the reaction is carried out at a temperature comprised between 100 °C and 180 °C.

When R¹ of the intermediate of formula (II) is an intermediate form that can be transformed to a tetrazolyl group, the process further comprises the conversion of said intermediate form to a tetrazolyl group. In one embodiment, said intermediate form is a cyano group. But other intermediate forms that can be transformed to a tetrazolyl group may be used, such as an hydrazinoiminomethyl group. These intermediate forms may be converted to the tetrazole by methods known by the skilled in the art. For instance, when it is a cyano group, it can be transformed by several procedures using hydrazoic acid (e.g. by heating sodium azide and ammonium chloride as described in J. P. Hurwitz y A. J. Tomson, J. Org. Chem., (1961), 26, 3392). Preferably the tetrazole is prepared by the 1,3-dipolar cycloaddition of trialkyltin or triaryltin azides to the nitrile, as described in e.g. EP475898 or WO9906398.

When R¹ of the intermediate of formula (II) is a protected form that can be transformed to a tetrazolyl group, and G is H, the process comprises a further step in which the protective group is cleaved from the tetrazole ring. The protective group of the tetrazole ring can be removed by procedures known in the art (cf. Protective Groups in Organic Synthesis, Wiley-Interscience, (1999)). For instance, when trityl group is used as the protective group of the tetrazole ring, it can be deprotected either in acidic or basic conditions. Preferably, the deprotection is carried out in acidic conditions, for example, HCl in a suitable solvent such as methanol or a mixture of dioxane/water.

Preferably, the process according to the first aspect of the invention is carried out without the use of protecting groups. Thus, in a preferred embodiment G is H and R¹ is tetrazole.

The compound of formula (I) obtained by the process according to the first aspect of the invention, may be converted to a pharmaceutically acceptable salt thereof by methods well known to the skilled in the art.

The intermediate of formula (III) may be prepared by methods described in the literature (c.f. Winters, G. et al., Farmaco, Edizione Scientifica (1966), 21 (9), 624-30). The method described therein comprises a two step process starting from cycloleucine that renders the product with a poor yield. The present inventors have found a new and simplified method that renders the intermediate of formula (III) in one step, with high yields. Thus, a separate novel aspect of the invention relates to a process for preparing an intermediate of formula (III) wherein it is prepared by reaction between cycloleucine and valeroyl chloride. The reaction is carried out in an appropriate solvent system, preferably in aprotic solvents, such as toluene or tetrahydrofuran (THF), and in presence of a base able to capture the hydrochloric acid liberated during the reaction, such as trialkylamines, Hünig's bases or inorganic bases such as carbonates or hydroxides, preferably triethylamine or diisopropylethylamine. It may be carried out at a temperature comprised between 20 and 150°C, preferably between 50 and 110°C.

In a preferred embodiment of the first aspect of the invention, the intermediate of formula (III) is prepared according to the above mentioned separate aspect of the invention.

The intermediate of formula (II) may be prepared by several methods described in the literature, like those described in WO9906398 , WO9308169, WO9316049, EP540356 and EP542554.

The present inventors have also identified a new process for the preparation of intermediate of formula (II) by a Suzuki coupling reaction. Thus, a separate novel aspect of the invention relates to a process for preparing an intermediate of formula (II) wherein it is prepared by reaction between an intermediate of formula (IV) 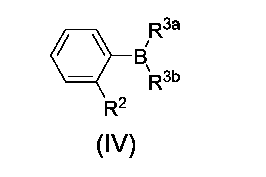 wherein:
R² is a tetrazolyl group or an intermediate or protected form that can be transformed to a tetrazolyl group,
R^{3a} and R^{3b} are each independently selected from the group consisting of: Cl, Br, (C₁-C₄)-alkoxy, hydroxy, or alternatively,
R^{3a} and R^{3b} can be taken together with the B atom to form a cyclic structure selected from the following ones 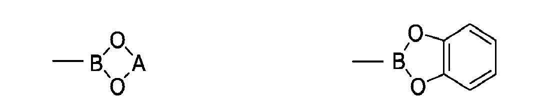
wherein A is (CH₂)ₙ and n is an integer from 2 to 4,
and an intermediate of formula (V) or an acid addition salt thereof 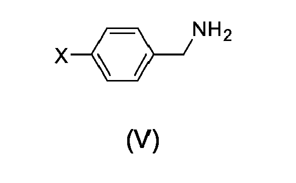 wherein:
X is a leaving group,
in the presence of a base, a metallic catalyst and an appropriate solvent system and optionally transforming said intermediate or protected form of R² to a tetrazolyl group and if desired converting the compound of formula (II) to an acid addition salt thereof.

In a preferred embodiment of the first aspect of the invention, the intermediate of formula (II) is prepared according to the above mentioned separate aspect of the invention.

Different solvent systems may be appropriate for the reaction. Preferably, the solvent system is selected from water, an organic solvent and mixtures of water and one or more organic solvents. Preferably, the solvent is selected from C₁-C₄ alcohols, DMF, DME, THF, and their mixtures with water. In a more preferred embodiment, the solvent system is selected from the group consisting of DMF and DME.

A variety of bases may be used in the process. Suitable bases may be selected from organic and inorganic bases. Preferably, the base is selected from alkaline hydroxides and alkaline carbonates. More preferably, the base is selected from sodium hydroxide and potassium hydroxide.

The suitable metallic catalysts include catalysts of palladium (0) or nickel, such as, tetrakis(triphenylphosphine)palladium (0), Bis(triphenylphosphine)palladium (II) dichloride, a complex formed by palladium acetate or palladium chloride or Pd/C with triaryl or trialkylphosphines optionally substituted, (1,3-bis[diphenylphosphino]propane)dichloronickel (II) (Ni(dppp)Cl₂), dichloro[1,1'-bis(diphenylphosphino)ferrocene]nickel (II) (Ni(dppf)Cl₂). Preferably, the metallic catalyst is selected from tetrakis(triphenylphosphine)palladium (0), a complex formed by palladium chloride with triphenylphosphine.

Preferably, the leaving group X is selected from an atom of halogen (Cl, Br, I), and a trifluoromethanesulfonyloxy group.

In a preferred embodiment, R^{3a} and R^{3b} are hydroxy and R² is a tetrazolyl group.

Suitable protecting groups for the tetrazole ring, procedures for introducing them and removing them are described in Greene and Wuts (Protective Groups in Organic Synthesis, Wiley and Sons, 1999).

In a preferred embodiment R¹ is a tetrazolyl group.

Intermediate of formula (IV), when R¹ is a tetrazolyl group may be obtained as described in the literature (e.g. according to example II of DE4313747 patent).

In a still more preferred embodiment, the intermediate of formula (IIa), 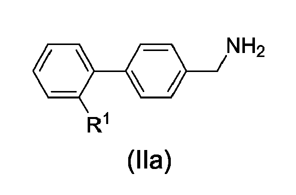 wherein R¹ is a tetrazolyl group or a protected form that can be transformed to a tetrazolyl group, is prepared in a "one-pot" process from an intermediate of formula (VI) 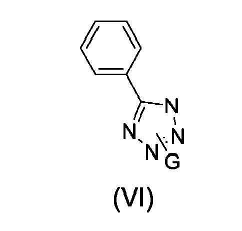 wherein G is as defined above. Thus, the process for preparing the intermediate of formula (IIa), according to the embodiment described above, in a still more preferred embodiment, further comprises previously preparing "in situ" an intermediate of formula (IV), by reaction of an intermediate of formula (VI), with an alkyllithium compound of formula R⁴-Li wherein R⁴ is a C₁-C₆ linear or branched alkyl and a boronic ester of formula B(OR⁵)₃ wherein R⁵ is a (C₁-C₄)-alkyl group.

This reaction may be carried out in anhydrous aprotic solvents, such as THF, diethylether or 1,2-dimethoxyethane.

Preferably, the alkyllithium compound R²-Li is selected from hexyllithium and butyllithium. Preferably, R⁵ in the boronic ester is a methyl or isopropyl group. Preferably, G in the compound of formula (VI) is H.

If desired, intermediate of formula (II) may be converted to an acid addition salt thereof. The addition salts, where applicable, can be prepared by treatment with acids, such as hydrochloric, hydrobromic, sulphuric, nitric, phosphoric, alkyl or arylsulfonic, in water or organic solvents such as ethers, alcohols, ketones, esters, or mixtures of solvents.

Throughout the description and claims the word "comprise" and variations of the word, such as "comprising", are not intended to exclude other technical features, additives, components, or steps. Additional objects, advantages and features of the invention will become apparent to those skilled in the art upon examination of the description or may be learned by practice of the invention. The following examples are provided by way of illustration, and are not intended to be limiting of the present invention.

### EXAMPLES

### EXAMPLE 1

### (2'-(1H-tetrazol-5-yl)biphenyl-4-yl)methanamine hydrochloride

0.42 g (2.21 mmol) of 2-(1*H*-tetrazol-5-yl)phenylboronic acid (obtained according to example II of DE4313747 patent), 0.49 g (2.20 mmol) of (4-bromophenyl)methanamine hydrochloride, 0.53 g (13.25 mmol) of sodium hydroxide and 0.13 g (0.112 mmol) of tetrakis(triphenylphosphine)palladium (0) were added to a mixture of 5 mL of DMF and 0.5 mL of water. Nitrogen was bubbled to the resultant mixture for 1 min and the reaction was heated to 100 °C under nitrogen atmosphere for 7 h. Once the DMF had been removed by distillation under reduced pressure, 5 mL of water were added and the mixture was washed twice using 5 mL of ethyl acetate each time. The aqueous phase was adjusted to pH 1 with concentrated hydrochloric acid and washed again three times with 5 mL of ethyl acetate. Remains of ethyl acetate were distilled from the aqueous phase and the mixture was left to precipitate for 1 h at room temperature and later for 1 h at 0 °C. The solid was filtered, washed with cold water and dried under reduced pressure at 60 °C affording (2'-(1*H*-tetrazol-5-yl)biphenyl-4-yl)methanamine hydrochloride.

RMN ¹H (DMSO), δ(ppm): 4.00 (d, 2H, Ar-CH₂-); 7.14 (d, 2H, ArH); 7.40 (d, 2H, ArH); 7.52 (d, 1 H, ArH); 7.59 (d, 1 H, ArH); 7.65-7.72 (m, 2H, ArH); 8.37 (sa, 3H, -NH₃⁺C⁻).

M.P. = 283 °C (decomposes)

### EXAMPLE 2

### (2'-(1H-tetrazol-5-yl) biphenyl-4-yl) methanamine hydrochloride

Under nitrogen atmosphere, 6.6 mL of 1,2-dimethoxyethane were added to 0.66 g (4.52 mmol) of 5-phenyl-1*H*-tetrazole. The suspension was cooled to 0 °C and then 4.7 mL (10.81 mmol) of 2.3 M solution of hexyllithium in hexanes was slowly added maintaining the temperature at between 0 and 10 °C. After 1 h at 0 °C, 1.1 mL (9.81 mmol) of trimethyl borate was added and the reaction was left stirring at room temperature for a further 2 h. One mL of water, 1.08 g (27 mmol) of sodium hydroxide, 1 g (4.49 mmol) of (4-bromophenyl)methanamine hydrochloride and 0.208 g (0.18 mmol) of tetrakis(triphenylphosphine)palladium (0) were successively added and the mixture was heated to reflux under nitrogen atmosphere for 5 h. Afterwards, 5 mL of water were added and the resultant aqueous phase was washed three times with 5 mL of ethyl acetate. The aqueous phase was adjusted to pH 1 with concentrated hydrochloric acid and washed again three times with 5 mL of ethyl acetate. Remains of ethyl acetate were distilled from the aqueous phase and the mixture was left to precipitate for 1 h at room temperature and later for 1 h at 0 °C. The solid was filtered, washed with cold water and dried under reduced pressure at 60 °C affording 0.64 g of a crude that can be crystallized from 2.5 mL of water to obtain pure (2 '-(1*H*-tetrazol-5-yl) biphenyl-4-yl) methanamine hydrochloride.

### EXAMPLE 3

### 2-n-butyl-3-oxa-1-azaspiro-[4.4]-non-1-en-4-one

9.2 mL (77.5 mmol) of valeroyl chloride were added to a suspension of 5 g (38.7 mmol) of 1-aminocyclopentanecarboxylic acid in 50 mL of toluene. The mixture was heated to 80 °C and 11.9 mL (85.4 mmol) of triethylamine were slowly added over one hour. The reaction was left for 4 h at 80 °C and, after cooling to room temperature, was washed twice with 50 mL of water, twice with 25 mL of 10 % aqueous potassium carbonate solution, once with 25 mL saturated ammonium chloride solution and finally with 25 mL of water. The resultant organic phase was evaporated at a pressure of 200 mbar to obtain 10.16 g of yellowish oil that was purified by distillation at 10 mbar (130-140 °C) affording 5.62 g (74.4 %) of 2-n-butyl-3-oxa-1-azaspiro-[4.4]-non-1-en-4-one.

RMN ¹H (CDCl₃), δ(ppm): 0.94 (t, 3H, -CH₂-CH₂-CH₂-CH₃); 1.32-1.47 (m, 2H, -CH₂-CH₂-CH₂-CH₃); 1.61-1.73 (m, 2H, -CH₂-CH₂-CH₂-CH₃); 1.82-2.08 (m, 8H, cyclopentane); 2.46 (t, 2 H, -CH₂-CH₂-CH₂-CH₃).

### EXAMPLE 4

### 2-n-butyl-3-[[2'-(tetrazol-5-yl)biphenyl-4-yl]-methyl]-1,3-diazaspiro[4.4]non-1-en-4-one

Under nitrogen atmosphere, a solution of 1.03 g (5.27 mmol) of 2-n-butyl-3-oxa-1-azaspiro-[4.4]-non-1-en-4-one in 1 mL of N-methylpirrolidone was added over 30 min to a mixture of 1.0 g (3.47 mmol) of (2 '-(1*H*-tetrazol-5-yl) biphenyl-4-yl) methanamine hydrochloride in 3 mL of N-methylpirrolidone heated at 140 °C. The reaction was left for 5 h at 140 °C and 200 mbar and after cooling to room temperature 15 mL of 10 % aqueous sodium hydroxide solution was added. Then, 15 mL of ethyl acetate were added and the mixture was acidified with concentrated hydrochloric acid until pH 4-5. After a while, the mixture started to precipitate and after leaving for 1 h at 20-25 °C the solid was filtered, washed with water and ethyl acetate and, finally, the solid was dried under reduced pressure at 65 °C affording 0.90 g (60.4 %) of 2-n-butyl-3-[[2'-(tetrazol-5-yl)biphenyl-4-yl]-methyl]-1,3-diazaspiro [4.4] non-1-in-4-one.

RMN ¹H (CDCl₃), δ(ppm): 0.82 (t, 3H, -CH₂-CH₂-CH₂-CH₃); 1.18-1.33 (m, 2H, -CH₂-CH₂-CH₂-CH₃); 1.40-1.52 (m, 2H, -CH₂-CH₂-CH₂-CH₃); 1.62-1.86 (m, 8H, cyclopentane); 2.17 (t, 2 H, -CH₂-CH₂-CH₂-CH₃); 4.65 (s, 2H, Ar-CH₂-); 7.04 (d, 2H, ArH); 7.15 (d, 2H, ArH); 7.44 (dd, 1H, ArH); 7.49-7.65 (m, 2H, ArH); 7.87 (dd, 1H, ArH).

M.P. = 182°C

### EXAMPLE 5

### 2-n-butyl-3-[[2'-(tetrazol-5-yl)biphenyl-4-yl]-methyl]-1,3-diazaspiro[4.4]non-1-en-4-one

A solution of 0.44 g (2.25 mmol) of 2-n-butyl-3-oxa-1-azaspiro-[4.4]-non-1-en-4-one in 0.4 mL of N-methylpirrolidone was added over 10 min to a mixture of 0.4 g (1.39 mmol) of (2'-(1H-tetrazol-5-yl)biphenyl-4-yl)methanamine hydrochloride and 0.08 g (0.83 mmol) of methanesulfonic acid in 1.6 mL of N-methylpirrolidone heated at 160 °C. The reaction was left for 2.5 h at 160 °C and 200 mbar and after removing the N-methylpirrolidone by distillation under reduced pressure and cooling to room temperature 4 mL of 10 % aqueous sodium hydroxide solution was added. Then, 6 mL of ethyl acetate were added and the mixture was acidified with concentrated hydrochloric acid until pH 4-5. After a while, the mixture started to precipitate and after leaving for 1 h at 20-25 °C the solid was filtered, washed with water and ethyl acetate and, finally, the solid was dried under reduced pressure at 60 °C affording 0.42 g (70,5 %) of 2-n-butyl-3-[[2'-(tetrazol-5-yl)biphenyl-4-yl]-methyl]-1,3-diazaspiro[4.4]non-1-in-4-one.

## Claims

1. A process for preparing a compound of formula (I) or a pharmaceutically acceptable salt thereof 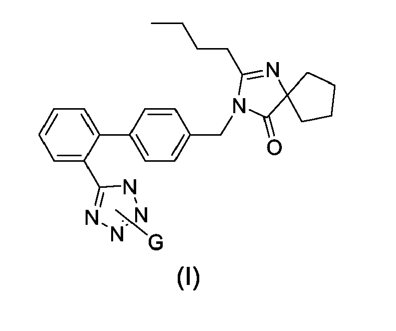 wherein:
G is H or a tetrazole protecting group,
comprising the reaction between an intermediate of formula (II) or an acid addition salt thereof 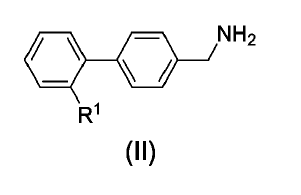
wherein:
R¹ is a tetrazolyl group or an intermediate or protected form that can be transformed to a tetrazolyl group
and an intermediate of formula (III) 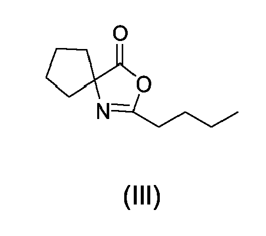
in an appropriate solvent system and thereafter as necessary transforming said intermediate or protected forms of R¹ to a tetrazolyl group and, if desired, converting said compound of formula (I) to a pharmaceutically acceptable salt thereof.

2. The process according to claim 1, wherein R¹ is a tetrazolyl group and G is H.

3. The process according to any of claims 1 or 2, wherein the reaction is carried out in the presence of an acid catalyst.

4. The process according to claim 3, wherein said acid catalyst is selected from the group consisting of: methanosulfonic acid, *p*-toluensulfonic acid, and hydrochloric acid.

5. The process according to any of claims 1 to 4, wherein said solvent system comprises a polar aprotic solvent.

6. The process according to any of the preceding claims, wherein the intermediate of formula (III) is prepared by reaction between cycloleucine and valeroyl chloride.

7. The process according to any of the preceding claims, wherein the intermediate of formula (II) or an acid addition salt thereof is prepared by reaction between an intermediate of formula (IV) 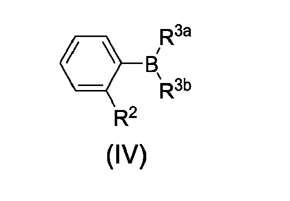 wherein:
R² is a tetrazolyl group or an intermediate or protected form that can be transformed to a tetrazolyl group,
R^{3a} and R^{3b} are each independently selected from the group consisting of: CI, Br, (C₁-C₄)-alkoxy, hydroxy, or alternatively,
R^{3a} and R^{3b} can be taken together with the B atom to form a cyclic structure selected from the following ones 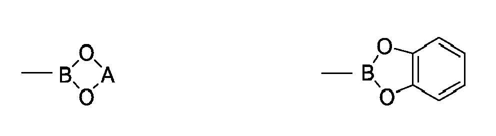
wherein A is (CH₂)ₙ and n is an integer from 2 to 4,
and an intermediate of formula (V) or an acid addition salt thereof 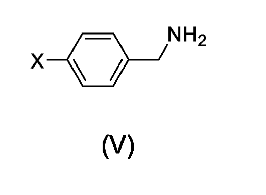 wherein:
X is a leaving group
in the presence of a base, a metallic catalyst and an appropriate solvent system and optionally transforming said intermediate or protected form of R² to a tetrazolyl group and if desired converting the compound of formula (II) to an acid addition salt thereof.

8. The process according to claim 7, wherein R^{3a} and R^{3b} are hydroxy and R² is a tetrazolyl group.

9. The process according to claim 7, that further comprises previously preparing "in situ" an intermediate of formula (IV), by reaction of an intermediate of formula (VI) 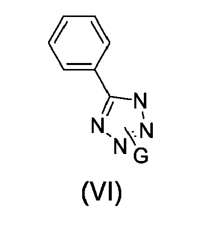 wherein G is as defined above,
with an alkyllithium compound of formula R⁴-Li, wherein R⁴ is a C₁-C₆ linear or branched alkyl, and a boronic ester of formula B(OR⁵)₃, wherein R⁵ is a (C₁-C₄)-alkyl group.

10. The process according to claim 9, wherein G is H.
